Europäisches Patentamt

(19) European Patent Office

Office européen des brevets

(11) Numéro de publication: **0 242 267**
**B1**

(12) **FASCICULE DE BREVET EUROPEEN**

(45) Date de publication du fascicule du brevet:
08.11.89

(21) Numéro de dépôt: **87400761.0**

(22) Date de dépôt: **06.04.87**

(51) Int. Cl.⁴: **A61B 17/58, A61F 2/28**

(54) **Ensemble de plaque de fixation osseuse, muni de fils de suture.**

(30) Priorité: **10.04.86 FR 8605146**

(43) Date de publication de la demande:
**21.10.87 Bulletin 87/43**

(45) Mention de la délivrance du brevet:
**08.11.89 Bulletin 89/45**

(84) Etats contractants désignés:
**AT BE CH DE ES FR GB GR IT LI LU NL SE**

(56) Documents cités:
**EP-A- 0 014 823**
**EP-A- 0 052 998**
**EP-A- 0 065 098**
**US-A- 3 730 187**

**ENGINEERING IN MEDICINE,**
**vol. 9, no. 3, juillet 1980, pages 127-130, MEP Ltd,**
**Londres, GB; J.S. BRADLEY et al.: "Carbon fibre**
**reinforced plastics for load-bearing implants"**

(73) Titulaire: **Peters, 11 bis rue Chapon,**
**F-93300 Aubervilliers(FR)**

(72) Inventeur: **Popoff, Georges, 13 rue Moliveau,**
**F-75005 Paris(FR)**
Inventeur: **Schwarz, Georg Erwin, 9 rue Joliot Curie,**
**F-95170 Goussainville(FR)**

(74) Mandataire: **Tanguy, Gilbert André et al, c/o**
**Harlé-Phélip 21, rue de la Rochefoucauld,**
**F-75009 Paris(FR)**

## Description

La présente invention appartient au domaine des instruments et articles à usage médical et/ou chirurgical. Elle a pour objet une plaque de fixation osseuse munie de fils de suture et pouvant être mise en place à demeure en contact ou en substitut d'une partie osseuse du corps humain ou animal. L'invention trouve une application particulièrement intéressante dans le domaine de la sternotomie, qui servira ci-après à illustrer, à titre d'exemple, le problème qui est à la base de la présente invention.

Dans de nombreuses opérations chirurgicales où le chirurgien est obligé d'intervenir dans la cage thoracique, par exemple lors d'opérations sur les poumons, sur le coeur ou sur les grosses artères, il est contraint d'ouvrir le thorax afin d'avoir accès aux organes. Cette ouverture se fait, en général, par une sternotomie verticale. Après cette opération, le chirurgien est forcément obligé de refermer la sternotomie. Pour cela, il utilise des fils de suture individuels, sertis en général sur des aiguilles courbes. Pour chaque fermeture du sternum, il emploie entre 3 et 6 fils suivant le volume du thorax ou la longueur du sternum.

Actuellement, en France, les chirurgiens cardio-vasculaires procèdent chaque année à environ 30 000 interventions nécessitant un abord par sternotomie verticale. A chaque intervention de ce genre, les chirurgiens utilisent des fils du type précité pour fermer le thorax, une fois que l'intervention est terminée. Les fils passés autour ou à travers le sternum comme un cerclage servent à maintenir les deux berges de l'os stables afin que puisse s'effectuer la consolidation naturelle qui demande plusieurs semaines.

Une des complications constatées à ce stade, est la désunion des deux berges du sternum pouvant aboutir à la pseudarthrose qui peut, ou non, être infectée.

Il arrive, en outre, surtout chez les personnes âgées, que les fils de suture, qui sont constitués de gros fils solides, causent aux os, en particulier au sternum, de sérieux dommages, qui peuvent être irréversibles en raison du caractère friable de ces tissus.

L'utilisation de fils de suture individuels, sans liaison entre eux, peut donc s'accompagner d'inconvénients mécaniques graves.

D'autre part, tous les malades ayant bénéficié d'une intervention par sternotomie verticale sont soumis, dans les mois ou les années suivantes, au risque d'une réintervention par la même voie d'abord. Or, après la première intervention, des adhérences se créent entre la face postérieure du sternum et les organes voisins. Ces organes peuvent être les plèvres ou les poumons. Le péricarde s'il a pu être rapproché, les gros vaisseaux du médiastin, en particulier le tronc veineux innominé ou l'aorte, ou les éléments antérieurs du coeur lui-même (oreillette droite ou ventricule droit). Tous ces organes peuvent venir s'accoler de façon intime au sternum.

On comprend donc que, dans ces conditions, la réouverture du sternum par la scie oscillante lors de la ou des réinterventions, comporte un danger rare mais imprévisible d'effraction d'organe avec risque d'hémorragie cataclismique.

Lors de la première intervention, ce phénomène ne se produit pas, car le coeur et les gros vaisseaux qui en sortent sont enveloppés dans le péricarde (membrane fibreuse) que l'on peut écarter facilement.

Le brevet EP-A-0 014 823 (Howmedica) décrit une barrette de faible longueur (maximum 80 mm) pouvant être fixée sur les côtes pour maintenir celles-ci en cas de fracture ou de détérioration. La barrette est munie à cet effet de crampons pointus qui s'enfoncent dans les côtes. Elle fait donc uniquement fonction d'attelle. Les caractéristiques d'une telle barrette la rendent impropre à l'utilisation comme plaque de fixation osseuse, en particulier pour le sternum après sternotomie. Par ailleurs ladite barrette connue ne comporte pas de fils de suture. Ainsi le brevet EP-A-0 014 823 ne permet pas de résoudre le problème technique consistant à réaliser une structure assurant à la fois la fixation osseuse et une mise en place correcte des fils de suture, de manière à éviter l'effrittement des os sollicités (tels que le sternum) et à protéger les organes sous-jacents, notamment en cas de nouvelle intervention chirurgicale.

Le brevet US-A-3 730 187 (Reynolds) illustre un cathéter pour drainage urétral, qui est muni à l'avance d'un fil de suture pour permettre son maintien. Il est tout-à-fait clair qu'un tel dispositif ne permet pas de résoudre le problème technique qui vient d'être mentionné.

La présente invention a pour objet un ensemble de plaque pour fixation osseuse, principalement du sternum après sternotomie, qui apporte une solution à un tel problème. Cette plaque fait fonction d'implant et non de prothèse. Elle facilite en outre le travail du chirurgien, grâce au fait qu'elle est munie de fils maintenus séparément sur lesquels les aiguilles sont serties, l'ensemble étant conditionné dans un emballage stérile.

La présente invention a encore pour objet un ensemble de plaque pouvant être mis en place à demeure, en contact ou en substitution d'un os, par exemple le sternum, afin d'éliminer les inconvénients constatés dans l'art antérieur avec l'utilisation de fils de suture individuels.

L'invention concerne donc un ensemble de plaque pour la fixation osseuse comportant une plaque en matériau biocompatible, destinée à être mise en place au cours d'une intervention chirurgicale et à demeurer en contact ou en substitution d'un os, notamment dans le domaine de la sternotomie, caractérisé en ce que ladite plaque porte au moins un fil de suture dont les deux extrémités libres sont destinées à être liées entre elles pour maintenir la plaque en appui sur l'os.

L'ensemble de plaque conforme à l'invention, remplit plusieurs fonctions. Tout d'abord, la plaque mise en place sert de tuteur et rigidifie le montage avec l'os. Elle empêche en outre que les fils de suture endommagent l'os ou créent sur celui-ci des incidents irréversibles, notamment à cause de sa fragilité. Ces avantages sont particulièrement apparents lors d'une sternotomie. L'ensemble de plaque renforce la solidité de l'union des deux berges du sternum. La présence de la plaque empêche que les fils de suture scient la face postérieure du sternum par serrage excessif, ou en raison d'une fragilité éventuelle de l'os. En outre, la plaque mise en place exerce une fonction de protection sur les organes sousjacents. Dans l'exemple illustratif de la sternotomie, la plaque de l'invention, en s'interposant entre le sternum et les organes sous-jacents, élimine le danger en cas de réintervention, car le chirurgien, en sciant l'os, ne peut que rencontrer en premier lieu la plaque de séparation. L'ensemble de plaque exerce encore une autre fonction, du fait qu'il porte les fils de suture. Cela permet au chirurgien de disposer d'un certain nombre de fils, bien localisés et aisés à manipuler, contrairement à l'art antérieur où il était obligé de manipuler des fils individuels. Outre cette fonction de support des fils de suture, l'ensemble de plaque selon l'invention assure donc une plus grande facilité de manipulation au chirurgien.

Au sens de la présente description, l'expression "fils de suture" a le même sens que celui qu'on donne à cette expression dans la technique courante. L'invention présente le plus grand intérêt lorsque les fils de suture sont des gros fils à forte résistance mécanique, en acier inoxydable ou en autre matière, telle que le polyester.

La matière constitutive de la plaque selon l'invention n'est pas critique, mais il est indispensable qu'elle soit bio-compatible, c'est-à-dire qu'elle soit choisie parmi les matériaux ayant fait leurs preuves d'inocuité, de non toxicité et de non irritabilité; ainsi, la plaque peut être faite soit en matière métallique telle que l'acier inoxydable, le tantale, soit en matière plastique telle que le polyester, le polypropylène, le polyéthylène, le fluorure de polyvinylidène, le polytétrafluoréthylène, le polyhexafluoréthylène et autres matières analogues. Ces trois dernières matières ont la préférence, car elles sont connues et utilisées depuis un certain temps comme biomatériaux.

Les autres matières plastiques présentent un risque de dégradation à long terme et les plaques métalliques peuvent gêner en provoquant une résonance non désirée lors des examens R.M.N..

La forme de la plaque de l'invention n'est pas critique, elle devra être adaptée aux besoins locaux pour tenir compte des caractéristiques des os avec lesquels elle doit coopérer. Dans le cas où la plaque est mise en oeuvre en appui avec le sternum, elle a une forme générale rectangulaire allongée.

Il est avantageux que la plaque présente une certaine souplesse d'ensemble, bien que la résistance mécanique demeure un paramètre important. Ainsi, dans le cas de la sternotomie, la plaque doit être conçue pour épouser le galbe du thorax. Pour cette raison encore, les matières plastiques citées précédemment conviennent particulièrement bien.

L'autre constituant de l'ensemble de plaque selon l'invention consiste en au moins un fil de suture. Afin de solidariser les fils de suture avec la plaque et de faciliter la mise en place des sutures, dans un premier mode de réalisation, les fils passent à travers des trous respectifs prévus dans la plaque.

Les trous sont prévus de telle sorte que les fils puissent coulisser afin de se réajuster et de se centrer automatiquement lorsque le chirurgien fait son noeud dans le cas des fils en polyester ou entortille les deux brins du fil dans le cas des fils en acier inox.

Mais en variante, on peut aussi fixer les fils fermement sur la plaque. Dans ce cas, les fils de suture peuvent être fixés par soudure ou par collage, ou bien on peut encore les faire passer par des trous ou des chicanes de la plaque, de manière à ce qu'ils y soient maintenus.

Il va sans dire que le nombre de fils de suture dépend de la nature de l'intervention chirurgicale à réaliser. La distance des fils de suture entre eux est calculée de manière que le maintien en position et la solidarisation avec l'os soient optimaux.

Dans le cas d'une sternotomie, la plaque en biomatériaux est en général pourvue de 6 fils de suture. Ce nombre correspond à la quantité maximale que les chirurgiens utilisent habituellement.

Dans le cas d'un sternum plus court ou plus petit, par exemple lors d'une intervention sur une femme ou sur un enfant, le chirurgien peut sectionner à au moins un endroit prévu la plaque pour la raccourcir et pour enlever en même temps un ou deux fils dont il n'a plus besoin.

A cet effet, la plaque suivant l'invention peut être pourvue d'une ou plusieurs petites entailles permettant au chirurgien de sectionner aisément la longueur excessive.

Pour des raisons pratiques, les aiguilles doublement serties fixées sur le même fil sont repliées, reliées ensemble et maintenues en position par un tube en plastique ou par une mousse et, afin de ne pas emmêler les différents fils de suture et de faciliter la pose de la plaque lors de l'intervention, les fils repliés sont insolés les uns des autres par des feuilles ou des gaines en matière plastique.

L'invention sera maintenant illustrée, sans être aucunement limitée, par la description qui suit, qui concerne un ensemble de plaque utilisable en sternotomie, et ce en référence aux dessins annexés sur lesquels :

Fig. 1 représente en perspective l'ensemble de plaque avec deux fils de suture,

Fig. 2 est une vue analogue à la Fig. 1, montrant un fil de suture dans une gaine en matière plastique,

Fig. 3 est un schéma illustrant la mise en place de l'ensemble de plaque,

Fig. 4 est une coupe horizontale prise dans le plan IV-IV de la Fig. 3.

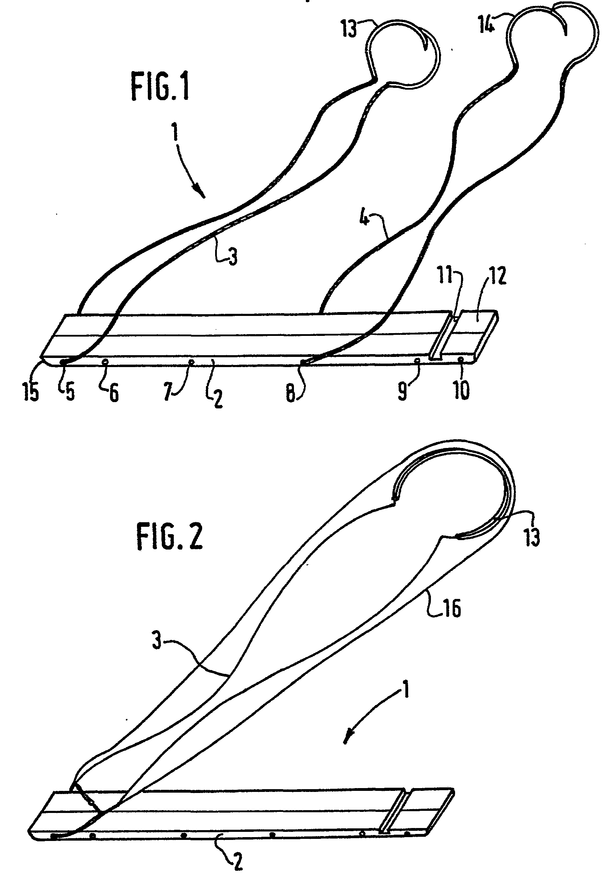

Tel qu'il est représenté à la Fig. 1, l'ensemble de plaque conforme à l'invention est désigné par la référence générale (1). Il comprend une plaque (2) de forme rectangulaire allongée. A titre d'exemple, cette plaque peut avoir une longueur de 20 cm et une largeur de 2 cm. Pour lui conférer une souplesse suffisante, son épaisseur est de 4 mm. La plaque (2) est munie d'un certain nombre de fils de suture dont on a seulement représenté deux d'entre eux, les fils (3,4). Ces fils sont en acier inoxydable et peuvent comporter à leurs extrémités des aiguilles courbes telles que (13, 14). Les fils de suture, dans l'exemple choisi, coulissent librement dans des ouvertures transversales de la plaque (2). Ces ouvertures portent les références (5, 6, 7, 8, 9 et 10), elles sont au nombre de six, dans l'exemple choisi. Pour simplifier la représentation du dessin , on a seulement représenté deux fils de suture (3, 4), qui passent respectivement dans les ouvertures (5, 8). Normalement, la plaque comporte six fils de suture.

A l'une des extrémités, la plaque (2) comporte une amorce de rupture (11) permettant, le cas échéant, au chirurgien de séparer la partie extrême (12), de manière à raccourcir la longueur de la plaque.

A l'autre extrémité, on a représenté en (15) une partie chanfreinée, de manière à se conformer le mieux possible aux nécessités chirurgicales.

Il va sans dire que l'exemple ci-dessus n'est qu'illustratif, l'invention n'est en aucun cas limitée à des formes de plaques rectangulaires. De même, les profils de la plaque peuvent être variés. On peut en outre conférer à celle-ci un galbe ou la renforcer en certains endroits, si cela s'avère nécessaire.

La Fig. 2 est une vue analogue à la figure 1, où l'on a représenté seulement un fil de suture (3). Par ailleurs, la plaque (2) est identique et il n'est donc pas nécessaire de reprendre toute la description la concernant. Le but de la figure 2 est d'illustrer le mode de réalisation selon lequel le fil de suture (3) est entouré d'une gaine plastique transparente (16), qui assure une protection des aiguilles (13). Dans la pratique, tous les jeux de fils de suture sont munis d'une telle gaine. Pour assurer la stérilité de l'ensemble, il est également prévu d'introduire l'ensemble de plaque avec ses fils de suture entourés respectivement d'une gaine de protection, au sein d'une enveloppe en matière plastique dont les bords ont été scellés. On répond ainsi aux nécessités chirurgicales de stérilisation.

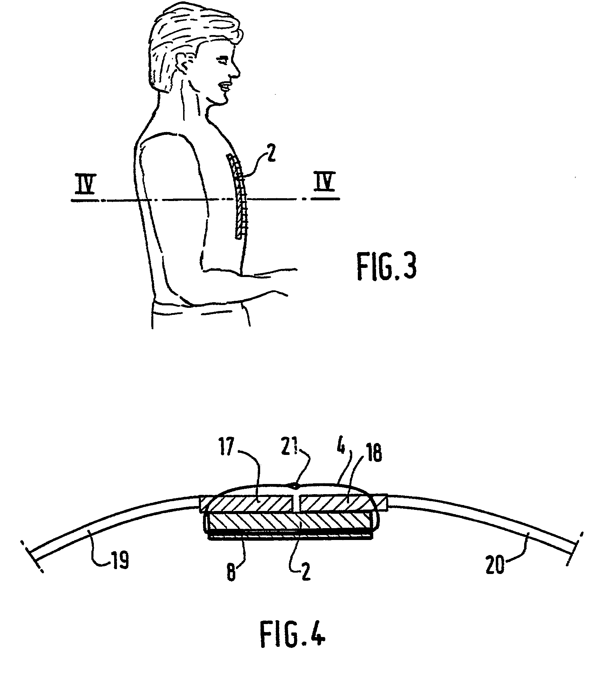

La Fig. 3 illustre d'une manière schématique le montage à demeure de la plaque (2) sur la partie antérieure du thorax d'un être humain ayant subi une sternotomie.

La Fig. 4 illustre avec davantage de précision la mise en place de l'ensemble de plaque (2). On voit que la plaque (2) vient en appui des deux parties (17, 18) du sternum qui ont été coupées lors de l'intervention. On a représenté en (19, 20) les côtes attachées au sternum à cet endroit. Un fil de suture, tel que (4), passe dans l'ouverture (8) de la plaque (2). Les extrémités libres du fil sont attachées comme représenté en (21). On notera que pour assurer une résistance optimale, la partie de la plaque (2) qui est en appui contre le sternum (17, 18) présente une épaisseur supérieure à celle de la partie opposée.

Les avantages de la mise en place de la plaque (2) ont été clairement mis en évidence dans l'introduction du présent mémoire. Il n'est donc pas utile d'y revenir maintenant dans la description d'un exemple spécifique. Une fois encore,celui-ci a été choisi pour les besoins de l'exposé, dans le domaine de la sternotomie. Il va sans dire, néanmoins, que l'invention est applicable à d'autres domaines chirurgicaux.

Par ailleurs, l'invention a été représentée aux dessins d'une façon schématique, dans le seul but de l'illustrer. L'omme du métier peut lui apporter des variantes sans pour autant sortir de son cadre. Ainsi, on a représenté une seule entaille sécable (11). Il va sans dire qu'on peut prévoir plusieurs de ces entailles, afin d'adapter la plaque aux dimensions corporelles.

**Revendications**

1. Ensemble de plaque pour fixation osseuse comportant une plaque (2) en matériau biocompatible, destiné à être mise en place au cours d'une intervention chirurgicale et à demeurer en contact ou en substitution d'un os, notamment dans le domaine de la sternotomie, caractérisé en ce que ladite plaque porte au moins un fil de suture (3, 4) dont les deux extrémités libres sont destinées à être liées entre elles pour maintenir la plaque en appui sur l'os.

2. Ensemble de plaque selon la revendication 1, caractérisé en ce que la plaque (2) est constituée d'un matériau biocompatible présentant des propriétés d'inocuité, de non toxicité et de non irritabilité, notamment d'une matière métallique, telle que l'acier inoxydable ou le tantale, ou bien d'une matière plastique telle que le polyester, le polypropylène, le polyéthylène, le fluorure de polyvinylidène, le polytétrafluoréthylène, le polyhexafluoréthylène et autres matières analogues.

3. Ensemble de plaque selon l'une des revendications 1 ou 2, caractérisé en ce que la plaque (2) a une forme adaptée aux caractéristiques des os avec lesquels elle doit coopérer.

4. Ensemble de plaque selon l'une quelconque des revendications 1 à 3, caractérisé en ce que la plaque (2) comporte au moins une partie sécable (12).

5. Ensemble de plaque selon l'une quelconque des revendications 1 à 4, caractérisé en ce que les fils de suture (3, 4) sont des gros fils à forte résistance mécanique en acier inoxydable ou en une autre matière, telle que le polyester.

6. Ensemble de plaque selon l'une quelconque des revendications 1 à 5, caractérisé en ce que les fils de suture (3, 4) passent à travers des trous respectifs (5, 6 ...10) prévus dans la plaque (2).

7. Ensemble de plaque selon l'une quelconque des revendications 1 à 5, caractérisé en ce que les fils de suture sont fixés fermement sur la plaque (2) par soudure ou par collage, ou bien en les faisant passer par des trous ou des chicanes de la plaque, de manière à ce qu'ils y soient maintenus.

8. Ensemble de plaque selon l'une quelconque des revendications 1 à 7, caractérisé en ce que les fils de suture (3, 4) portent des aiguilles recourbées (13, 14).

9. Ensemble de plaque selon l'une quelconque des revendications 1 à 8, caractérisé en ce que les aiguilles (13, 14) fixées sur le même fil (3) sont repliées, reliées ensemble et maintenues en position par un tube en plastique ou par une mousse et, fin de ne pas emmêler les différents fils de suture et de faciliter la pose de la plaque lors de l'intervention, les fils repliés sont isolés les uns des autres par des feuilles ou des gaines en matière plastique (16).

10. Ensemble de plaque selon l'une quelconque des revendications 1 à 9, caractérisé en ce qu'il est entièrement inséré dans une enveloppe en matière plastique dont les bords sont scellés, permettant ainsi la stérilisation.

**Claims**

1. Plate set for bone fixation comprising a plate (2) of biocompatible material intended to be put in place during a surgical operation and to remain in contact with or as a substitute for a bone, particularly in the field of sternotomy, characterized in thtat the said plate carries at least one suture strand (3, 4) whose two free ends are intented to be joined together to hold the plate resting against the bone.

2. Plate set according to Claiim 1, characterized in that the plate (2) consists of a biocompatible material having the properties of harmlessness, nontoxicity and nonirritability, paritcularly of a metal such as stainless steel or tantalum, or alternatively of a plastic such as polyester, polypropylene, polyethylene, polyvinylidene fluoride, polytetrafluoroethylene or polyhexafluoroethylene and other similar materials.

3. Plate set according to either of Claims 1 and 2, characterized in that the plate (2) has a shape adapted to the characteristics of the bones with which it is to interact.

4. Plate set according to any one of Claims 1 to 3, characterized in that the plate (2) comprises at least one sectionable portion (12).

5. Plate set according to any one of Claims 1 to 4, characterized in that the suture strands (3, 4) are thick sutures of high mechanical strength made of stainless steel or of another material such as polyester.

6. Plate set according to any one of Claims 1 to 5, characterized in that the suture strands (3, 4) pass through corresponding through corresponding holes (5, 6 ... 10) provided in the plate (2).

7. Plate set according to any one of Claims 1 to 5, characterized in that the suture strands are firmly secured to the plate (2) by welding or by adhesion, or alternatively by being threaded through holes or chicanes in the plate, so that they are held therein.

8. Plate set according to any one of Claims 1 to 7, characterized in that the suture strands (3, 4) carry curved needles (13, 14).

9. Plate set according to any one of Claims 1 to 8, characterized in that the needles (13, 14) secured to the same suture (3) are folded back, joined together and held in position by a plastic tube or by a foam, and the strands which are folded back are separated from each other by plastic sheets or sheaths (16) in order not to mix up the various suture strands and to aid the fitting of the plate during the operation (10).

10. Plate set according to any one of Claims 1 to 9, characterized in that it is inserted as a whole into a plastic enclosure whose ends are sealed, thus permitting sterilization.

**Patentansprüche**

1. Plattenvorrichtung für Knochenfixierung, die eine Platte (2) aus biokompatiblem Material aufweist, die durch einen chirurgischen Eingriff plaziert werden und in Berührung mit einem Knochen oder als Ersatz für einen Knochen verbleiben soll, insbesondere auf dem Gebiet der Sternotomie dadurch gekennzeichnet, daß die Platte mindestens einen Nähfaden (3, 4) trägt, dessen zwei freie Enden miteinander verbindbar sind, um die Platte in Anlage an dem Knochen zu halten.

2. Plattenvorrichtung nach Anspruch 1, dadurch gekennzeichnet, daß die Platte (2) aus einem biokompatiblen Material besteht, welches unschädlich, nichttoxisch und nichtirritierend ist, insbesondere aus einem metallischen Material, wie rostfreiem Stahl oder Tantal, oder aus einem Kunststoffmaterial wie Polyester, Polypropylen, Polyethylen, Polyvinylfluorid, Polytetrafluorethylen, Polyhexafluorethylen und ähnlichen Materialien.

3. Plattenvorrichtung nach Anspruch 1 oder 2, dadurch gekennzeichnet, daß die Form der Platte (2) den Charakteristika der Knochen angepaßt ist, mit denen sie zusammenwirken soll.

4. Plattenvorrichtung nach einem der Ansprüche 1 bis 3, dadurch gekennzeichnet, daß die Platte (2) mindestens einen abtrennbaren Abschnitt (12) aufweist.

5. Plattenvorrichtung nach einem der Ansprüche 1 bis 4, dadurch gekennzeichnet, daß die Nähfäden (3, 4) starke Fäden mit großer mechanischer Festigkeit aus rostfreiem Stahl oder einem anderen Material, wie Polyester, sind.

6. Plattenvorrichtung nach einem der Ansprüche 1 bis 5, dadurch gekennzeichnet, daß die Nähfäden (3, 4) quer durch jeweilige Löcher (5, 6, ... 10) verlaufen, welche in der Platte (2) vorgesehen sind.

7. Plattenvorrichtung nach einem der Ansprüche 1 bis 5, dadurch gekennzeichnet, daß die Nähfäden durch Löten oder Kleben fest auf der Platte (2) befestigt sind, oder daß sie derart durch Löcher oder Durchlaßorgane der Platte geführt sind, daß die Nähfäden in diesen gehalten sind.

8. Plattenvorrichtung nach einem der Ansprüche 1 bis 7, dadurch gekennzeichnet, daß die Nähfäden (3, 4) gekrümmte Nadeln (13, 14) tragen.

9. Plattenvorrichtung nach einem der Ansprüche 1 bis 8, dadurch gekennzeichnet, daß die an dem gleichen Faden (3) befestigten Nadeln (13, 14) von einem Kunststoffrohr oder einem Schaumstoffkörper zusammengefaßt, miteinander verbunden und in Position gehalten sind, und daß – damit die verschiedenen Nähfäden sich nicht verwirren und die Anbringung der Platte bei dem Eingriff erleichtert wird – die zusammengefaßten Fäden die einen von den anderen durch Folien oder Hüllen (16) aus Kunststoff isoliert sind.

10. Plattenvorrichtung nach einem der Ansprüche 1 bis 9, dadurch gekennzeichnet, daß die Vorrichtung vollständig in eine Hülle aus Kunststoff eingefügt ist, deren Ränder zur Sterilisation versiegelt sind.

1/2

FIG.1

FIG.2

FIG.3

FIG.4